# EUROPEAN PATENT SPECIFICATION

(11) **EP 4 243 845 B1**
(45) Date of publication and mention of the grant of the patent: **07.01.2026**
(21) Application number: 21811156.5
(22) Date of filing: 12.11.2021
(51) Int. Cl.: A61K 35/747, A61K 35/745, A61K 36/899, A61K 31/702, A61K 31/70, A61P 25/24, A61P 25/00, A61K 31/732, A61K 31/733

(54) **SYNBIOTIC COMPOSITION**
SYMBIOTISCHE ZUSAMMENSETZUNG
COMPOSITION SYMBIOTIQUE

(30) Priority: 12.11.2020 SE 2051326
(43) Date of publication of application: 20.09.2023
(73) Proprietor: Synbiotics AB, 234 40 Lomma (SE)
(72) Inventor: LAVEBRATT, Catharina, 192 73 SOLLENTUNA (SE); RÜEGG, Joëlle, 111 27 STOCKHOLM (SE); SCHALLING, Martin, 112 41 STOCKHOLM (SE)
(74) Representative: Ström & Gulliksson AB
(86) International application number: PCT/SE2021/051137
(87) International publication number: WO 2022/103320

(56) References cited:
- US-A1- 2007 286 916
- US-A1- 2011 091 431
- SKOTT ELIN ET AL: "Effects of a synbiotic on symptoms, and daily functioning in attention deficit hyperactivity disorder - A double-blind randomized controlled trial", BRAIN, BEHAVIOR AND IMMUNITY, ACADEMIC PRESS, SAN DIEGO, CA, US, vol. 89, 1 June 2020 (2020-06-01), pages 9 - 19, XP086288273, ISSN: 0889-1591, [retrieved on 20200601], DOI: 10.1016/J.BBI.2020.05.056
- WANG YING ET AL: "Probiotics and fructo-oligosaccharide intervention modulate the microbiota-gut brain axis to improve autism spectrum reducing also the hyper-serotonergic state and the dopamine metabolism disorder", PHARMACOLOGICAL RESEARCH, ELSEVIER, AMSTERDAM, NL, vol. 157, 17 April 2020 (2020-04-17), XP086154195, ISSN: 1043-6618, [retrieved on 20200417], DOI: 10.1016/J.PHRS.2020.104784

## Description

### Technical Field

The present disclosure relates to synbiotic composition, comprising probiotic bacterial strains and prebiotic fibres or oligosaccharides, for use in the treatment of a neuropsychiatric disorder or for use in the amelioration and/or reductions of symptoms originating from the neuropsychiatric disorder.

### Background art

During the last decades the importance of the gut microbiota for wellbeing and health has been intensively studied. New knowledge has resulted in a suggestion to view the gut microbiota as a new organ. Molecular genetic methods have revolutionized the insight into the composition of the microbiota and microbiota analysis has made it possible to include studies of the microbiota as part of many human intervention studies. One example where the microbiota has been shown to play an important role is on the gut-brain axis. The gut-brain axis is the bidirectional interaction between the gut and the central nervous system. Molecules of importance for this interaction are e.g. short chain fatty acids which can be produced by the gut microbiota.

Recent studies have reported that inflammatory condition often is linked to different diseases e.g. the metabolic syndrome and also to different neuropsychiatric conditions. Prevalence of inflammation in psychiatric disorders is estimated to 20-40%. Studies of the gut microbiota in humans with different neuropsychological conditions have been conducted recently and the studies show that the microbiota is less diverse in those groups of individuals than in healthy individuals.

Currently, many neuropsychiatric conditions are treated with pharmacological substances. Such pharmacological treatments are often accompanied by several adverse effects. Thus, there is a need for alternative or complementary treatments of neuropsychiatric disorders/conditions.

Skott et al. (Brain, Behaviour and Immunity, vol. 89 (2020), pages 9-19) relates to the effects of a synbiotic composition on symptoms and daily functioning in ADHD.

US 2011/091431 A1 relates to compositions and methods comprising *Pediococcus* for reducing at least one symptom associated with autism spectrum disease (ASD).

### Summary

According to a first aspect, the above and other objects of the invention are achieved, in full or at least in part by a composition as defined by claim 1. According to this claim, the above object is achieved by a synbiotic composition for use in the treatment of a neuropsychiatric disorder or for use in the amelioration and/or reduction of symptoms originating from the neuropsychiatric disorder, wherein the neuropsychiatric disorder is chosen from the group consisting of ADHD, ADD, Asperger's syndrome and autism; and wherein the synbiotic composition comprises a *Lactobacillus paracasei* strain, wherein the *Lactobacillus paracasei* strain is *Lactobacillus paracasei* (LMG P-17806) or *Lactobacillus paracasei* (LMG P-26118), *Pediococcus pentosaceus* (LMG P-*20608), Lactobacillus plantarum* (LMG P-20606), and *Leuconostoc mesenteroides* (LMG P-20607); and at least one dietary fiber chosen from the group consisting of inulin, pectin, beta-glucan, resistant starch, a galacto-oligosaccharide, an isomalto-oligosaccharide, and a rice fiber.

According to one embodiment, the composition further comprises at least one *Bifidobacterium breve* strain, preferably *Bifidobacterium breve* (LMG P-26117).

According to another embodiment, the *Lactobacillus paracasei* strain is *Lactobacillus paracasei* (LMG P-17806).

According to one embodiment, the composition comprises inulin, pectin, beta-glucan and resistant starch.

The composition may comprise resistant starch, a galacto-oligosaccharide, and/or an isomalto-oligosaccharide.

The composition may comprise a rice fiber.

The composition may comprise inulin, pectin, beta-glucan resistant starch and/or rice fibers.

According to yet another embodiment, the symptoms of the neuropsychiatric disorder are chosen from the group consisting of ADHD symptoms as measured by the Attention-Deficit/Hyperactivity Disorder Rating Scale), inattention, and hyperactivity.

According to a further embodiment, the symptoms of the neuropsychiatric disorder are chosen from the group consisting of difficulty in emotion regulation, perceived stress, and obsessions and compulsions as measured by the Yale-Brown Obsessive Compulsive Scale.

According to one embodiment, the total amount of bacteria in one dose is 1×10⁶ to 1×10¹³, such as 5×10⁶ to 1×10¹³, such as 1×10⁷ to 1×10¹³, such as 5×10⁷ to 1×10¹³, such as 1×10⁸ to 1×10¹³, such as 5×10⁸ to 5×10¹², especially 1×10⁹ to 1×10¹², such as 5×10⁹ to 9×10¹¹, such as 1×10¹⁰ to 8×10¹¹, such as 5×10¹⁰ to 7×10¹¹, such as 1×10¹¹ to 6×10¹¹, such as 3×10¹¹ to 5×10¹¹, such as 4×10¹¹ CFUs.

According to a further embodiment, the total amount of fibers in one dose is between 0.1 to 20 g, such as 0.5 to 20 g, such as 1 to 20 g, such as 2 to 15 g, such as 2.5 to 12.5 g, such as 5 to 10 g, such as 6 to 8.5 g, such as 7.5 g.

According to a further embodiment, the total amount of bacteria in one dose is 4×10¹¹ CFUs and the total amount of fibers in one dose is 10 g.

According to one embodiment, the synbiotic composition is administered orally or rectally or by tube feeding.

According to another embodiment, the synbiotic composition is administered 1 to 3 times a day, 1 to 7 times a week.

According to a further embodiment, the synbiotic composition is in the form of a powder, a capsule, a tablet, a lozenge, a liquid, an emulsion, an enema, a suppository, or a tube feeding; and/or the synbiotic composition is a food supplement, a food product, a nutritional supplement, a natural remedy or a pharmaceutical product.

The food product may be a biscuit, a cracker, a bar, a liquid, e.g. a shot.

The bacterial strains disclosed here in have previously been deposited with the Belgian Coordinated Collections of Micro-organisms (BCCM), Laboratorium voor Microbiologie Bacteriënverzameling (LMG), Universiteit Gent, K.L. Ledenganckstraat 35, 9000 Gent, Belgium (Web site: http://bccm.belspo.be).

*Pediococcus pentosaceus* (LMG P-20608), *Lactobacillus plantarum* (LMG P-20606), and *Leuconostoc mesenteroides* (LMG P-20607) were deposited on June 19, 2001.

*Lactobacillus paracasei* (LMG P-17806) has been deposited with the Belgian Coordinated Collection of Microorganisms.

*Lactobacillus paracasei* (LMG P-26118) and *Bifidobacterium breve* (LMG P-26117) were deposited on November 24, 2010.

*Pediococcus pentosaceus* (LMG P-20608), *Lactobacillus plantarum* (LMG P-20606), *Lactobacillus paracasei* (LMG P-17806) and *Leuconostoc mesenteroides* (LMG P-20607) have previously been published in EP 1 624 762 B1.

*Lactobacillus paracasei* (LMG P-26118) and *Bifidobacterium breve* (LMG P-26117) have previously been published in EP 2 672 980 B1.

Other objectives, features and advantages of the present disclosure will appear from the following detailed description, from the experimental data, as well as from the attached claims. It is noted that the disclosure relates to all possible combinations of features.

Generally, all terms used in the claims are to be interpreted according to their ordinary meaning in the technical field, unless explicitly defined otherwise herein. All references to "a/an/the [component, means, step, etc.]" are to be interpreted openly as referring to at least one instance of said component, means, step, etc., unless explicitly stated otherwise. The steps of any method disclosed herein do not have to be performed in the exact order disclosed, unless explicitly stated.

As used herein, the term "comprising" and variations of that term are not intended to exclude other additives, components, integers or steps.

### Brief description of the drawings

Figs. 1a-1f shows the effect of a composition according to the present disclosure on different scores used in the assessment of different neuropsychological disorders.
Fig. 2a shows the responses from respondents who have used a composition according to the present disclosure for different time periods to the question "Do you feel that the synbiotic composition helped you with problems with depression/fatigue?".

### Detailed description

The gut with its microbiota has a key function to maintain immune homeostasis and approximately 70% of the immune system is estimated to be located in the gut.

Probiotics are defined as "a live microorganisms which, when administered in adequate amounts, confer a health benefit on the host" (FAO/WHO 2002).

Fibres are an essential part of our diet. Fibres can be divided into different groups and the group called soluble fibres are important for the gut microbiota. A group of the soluble fibres and oligosaccharides are called prebiotics.

Prebiotics are defined as "a substrate that is selectively utilized by host microorganisms conferring a health benefit" (International Scientific Association for Probiotics and Prebiotics 2017). When prebiotics are fermented in the gut, short chain fatty acids can be produced. Examples of the short chain fatty acids are acetic acid, butyric acid, propionic acid, and formic acid. It is known that short chain fay acids influence health, e.g. butyric acid nourishes the gut mucosa and propionic acid influences fat metabolism.

Synbiotics is defined as "an ingredient or dietary supplement combining probiotics and prebiotics for a synergy between the compounds".

The combination of strains presented herein and used in the studies and experiments were carefully selected to be optimal combination of strains for efficacy including bacterial survival and favourable influence on each other.

Different combinations of bacterial strains and fibers may be used in a composition according to the present disclosure. Different combinations of bacterial strains and different combinations of fibers are described below. Specific combinations of bacterial strains and fibers are shown in Table 1.

Throughout the present disclosure, specific bacterial strains are identified by their deposition number. Thus, "*Lactobacillus plantarum* (LMG P-20606)" can be denoted as "*Lactobacillus plantarum* (deposit no: LMG P-20606)" or "*Lactobacillus plantarum* LMG P-20606", all meaning a strain of *Lactobacillus plantarum* having the deposition number "LMG P-20606".

Compositions according to the present disclosure comprise a *Lactobacillus paracasei* strain, wherein the *Lactobacillus paracasei* strain is *Lactobacillus paracasei* (LMG P-17806) or *Lactobacillus paracasei* (LMG P-26118), *Pediococcus pentosaceus* (LMG P-20608), *Lactobacillus plantarum* (LMG P-20606), and *Leuconostoc mesenteroides* (LMG P-20607).

The composition may further comprise at least one *Bifidobacterium breve* strain. Preferably, the at least one *Bifidobacterium breve* strain is *Bifidobacterium breve* (LMG P-26117).

The composition may comprise *Lactobacillus plantarum* (LMG P-20606), *Lactobacillus paracasei* (LMG P-17806), *Pediococcus pentosaceus* (LMG P-20608) and *Leuconostoc mesenteroides* (LMG P-20607).

The composition may comprise *Lactobacillus plantarum* (LMG P-20606), *Lactobacillus paracasei* (LMG P-26118), *Pediococcus pentosaceus* (LMG P-20608) and *Leuconostoc mesenteroides* (LMG P-20607).

The composition may comprise *Lactobacillus plantarum* (LMG P-20606); *Lactobacillus paracasei* (LMG P-17806); *Pediococcus pentosacceus* (LMG P-*20608); Leuconostoc mesenterioides* (LMG P-20607); and *Bifidobacterium breve* (LMG P-26117).

The composition may comprise *Lactobacillus plantarum* (LMG P-20606); *Lactobacillus paracasei* (LMG P-26118); *Pediococcus pentosacceus* (LMG P-*20608); Leuconostoc mesenterioides* (LMG P-20607); and *Bifidobacterium breve* (LMG P-26117).

Compositions according to the present disclosure comprise at least one fiber chosen from the group consisting of inulin, pectin, beta-glucan, resistant starch, a galacto-oligosaccharide, an isomalto-oligosaccharide, and a rice fiber.

The composition may comprise resistant starch.

The composition may comprise inulin, pectin, beta-glucan and resistant starch.

The composition may comprise inulin, pectin, beta-glucan, rice fiber and resistant starch.

The composition may comprise resistant starch and a galacto-oligosaccharide.

The composition may comprise resistant starch and an isomalto-oligosaccharide.

The composition may comprise resistant starch, galacto-oligosaccharide and isomalto-oligosaccharide.

A composition according to the present invention may be administered one to three times a day.

Some examples of compositions according to the present invention as well as compositions outside the scope of the claims (compositions 1-18, 25-36 and 43-57) are shown in Table 1 below.

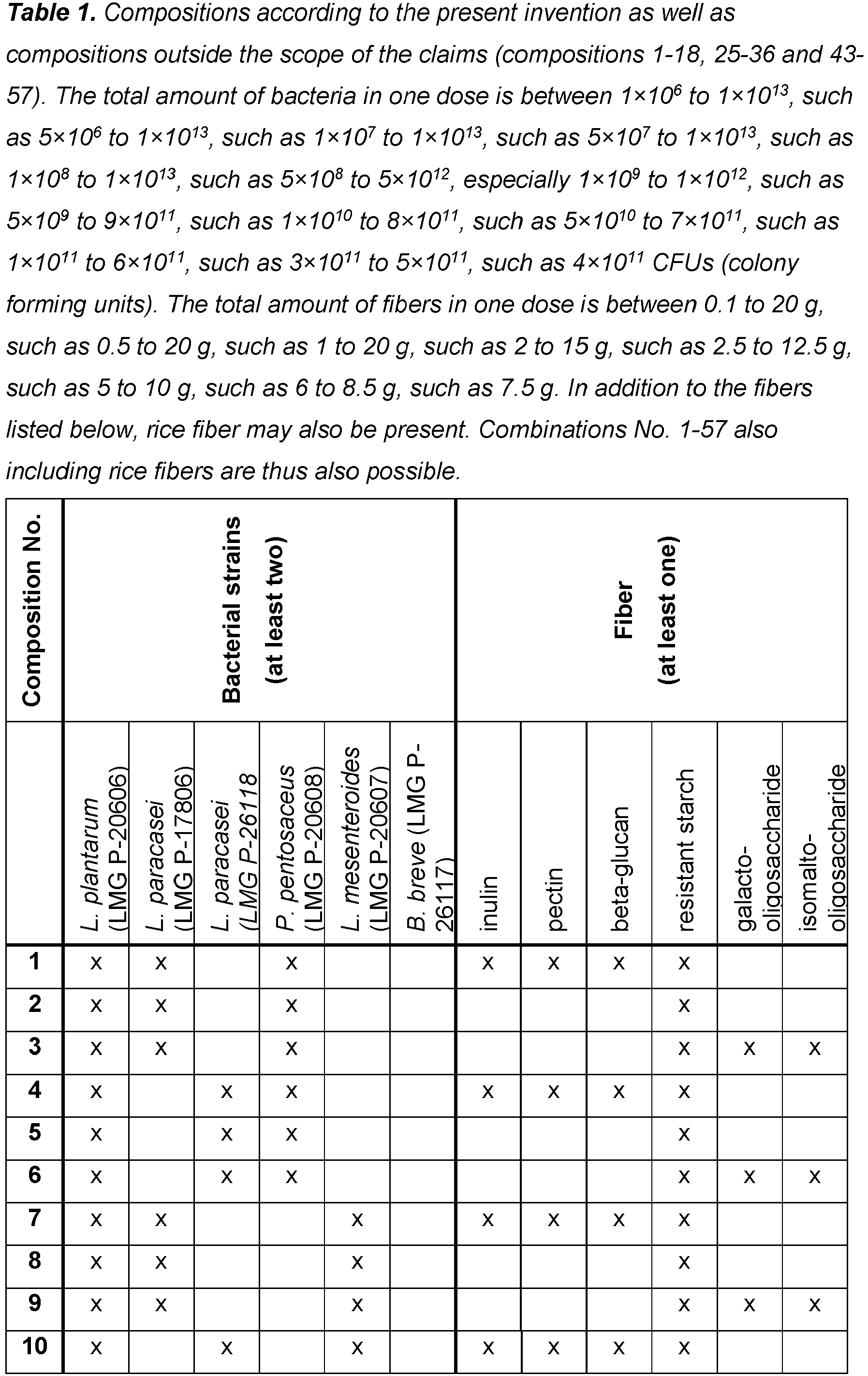

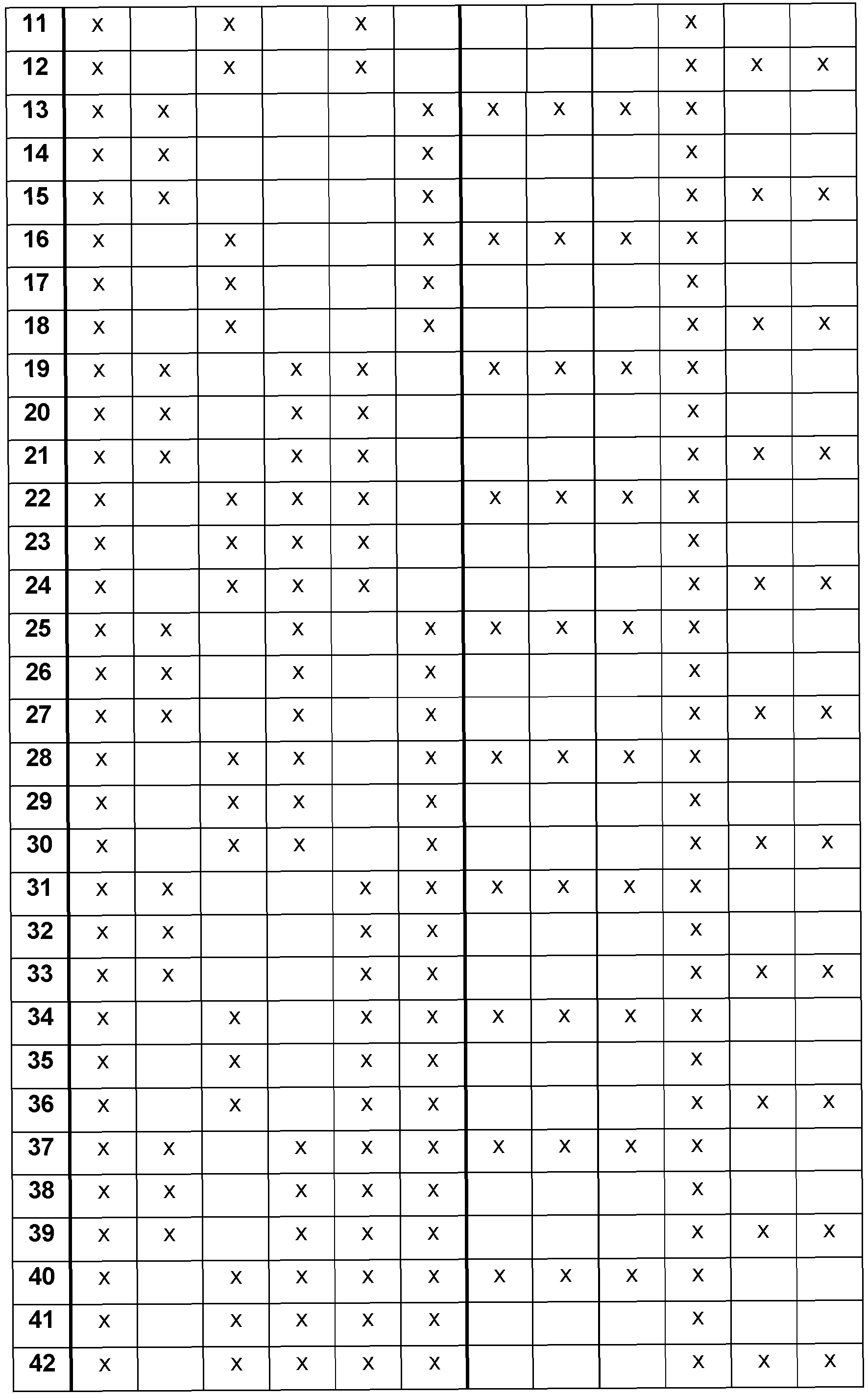

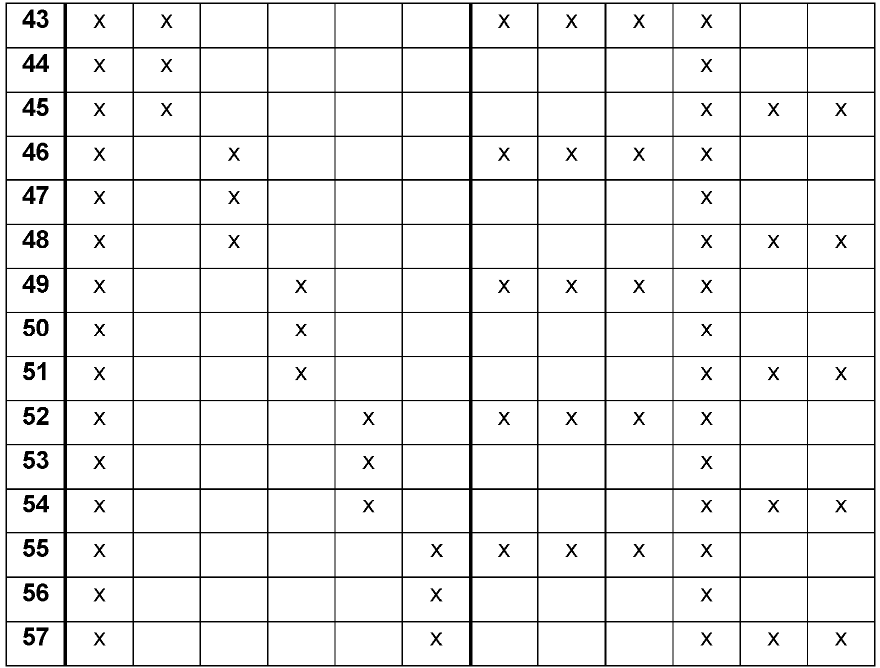

A composition according to the present invention may be administered one to seven days a week.

Preferably, a composition according to the present invention is administered once a day as a single dose.

The total amount of bacteria in one dose of a composition according to the present disclosure is between 1×10⁶ to 1×10¹³, such as 5×10⁶ to 1×10¹³, such as 1×10⁷ to 1×10¹³, such as 5×10⁷ to 1×10¹³, such as 1×10⁸ to 1×10¹³, such as 5×10⁸ to 5×10¹², especially 1×10⁹ to 1×10¹², such as 5×10⁹ to 9×10¹¹, such as 1×10¹⁰ to 8×10¹¹, such as 5×10¹⁰ to 7×10¹¹, such as 1×10¹¹ to 6×10¹¹, such as 3×10¹¹ to 5×10¹¹, such as 4×10¹¹ CFUs (colony forming units).

The total amount of fibers in one dose of a composition according to the present disclosure is between 0.1 to 20 g, such as 0.5 to 20 g, such as 1 to 20 g, such as 2 to 15 g, such as 2.5 to 12.5 g, such as 5 to 10 g, such as 6 to 8.5 g, such as 7.5 g.

A composition according to the present disclosure may be in the form of a dry powder intended to be mixed with a liquid, preferably water, before intake.

A composition according to the present disclosure may be in the form of a capsule, a tablet, a lozenge, a liquid, an emulsion, an enema, a suppository, or a tube feeding.

Patients suffering from a neuropsychiatric disorder such as ADHD, ADD, Asperger's syndrome and autism may be administered a composition according to the present invention in the form of a powder suspended in a liquid, preferably water, or in the form of a capsule, a tablet, a lozenge, a liquid, an emulsion, an enema, a suppository, or by tube feeding.

ADHD (attention deficit hyperactivity disorder) usually debuts in childhood and the worldwide prevalence of ADHD is 3-5%. Core symptoms are inattention and hyperactivity impulsivity. The disorder shows a great heterogeneity regarding manifestation and successful treatment. A large proportion of ADHD patients have psychiatric conditions e.g. mood disorders, anxiety disorders, learning disorders, and/or tic disorders, and also somatic disorders related to the immune system such as asthma, eczema, rhinitis and celiac disease.

The symptom(s) originating from ADHD that may be ameliorated and/or reduced may be one or more of symptoms chosen from the group consisting of inattention, hyperactivity, difficulties in emotion regulation, perceived stress and obsessive compulsive disorder. Specifically, the symptom is inattention and/or hyperactivity.

ADD (attention deficit disorder) usually also debuts in childhood. The core symptom is inattention. The disorder shows a great heterogeneity regarding manifestation and successful treatment. A large proportion of ADD patients have psychiatric conditions e.g. mood disorders, anxiety disorders, learning disorders, and/or tic disorders, and also somatic disorders related to the immune system such as asthma, eczema, rhinitis and celiac disease.

The symptom(s) originating from ADD that may be ameliorated and/or reduced may be one or more of symptoms chosen from the group consisting of inattention, difficulties in emotion regulation, perceived stress and obsessive compulsive disorder. Specifically, the symptom is inattention.

The symptom(s) originating from Asperger's syndrome and/or autism that may be ameliorated and/or reduced may be one or more of symptoms chosen from the group consisting of inattention, perceived stress and obsessive compulsive symptoms.

One composition that is specifically suitable for the treatment of ADHD, ADD, Asperger's syndrome and/or autism or for the amelioration or reduction of symptoms originating from ADHD, ADD, Asperger's syndrome and/or autism comprises *Lactobacillus plantarum* (LMG P-20606), *Lactobacillus paracasei* (LMG P-17806), *Pediococcus pentosaceus* (LMG P-20608) and *Leuconostoc mesenteroides* (LMG P-20607). Preferably, the composition also comprises *Bifidobacterium breve* (LMG P-26117).

Another composition that is specifically suitable for the treatment of ADHD, ADD, Asperger's syndrome and/or autism or for the amelioration or reduction of symptoms originating from ADHD, ADD, Asperger's syndrome and/or autism comprises *Lactobacillus plantarum* (LMG P-20606), *Lactobacillus paracasei* (LMG P-26118), *Pediococcus pentosaceus* (LMG P-20608) and *Leuconostoc mesenteroides* (LMG P-20607). Preferably, the composition also comprises *Bifidobacterium breve* (LMG P-26117).

A further composition that is specifically suitable for the treatment of ADHD, ADD, Asperger's syndrome and/or autism or for the amelioration or reduction of symptoms originating from ADHD, ADD, Asperger's syndrome and/or autism comprises *Lactobacillus plantarum* (LMG P-20606); *Lactobacillus paracasei* (LMG P-17806); *Pediococcus pentosacceus* (LMG P-20608); *Leuconostoc mesenterioides* (LMG P-20607); and *Bifidobacterium breve* (LMG P-26117).

A further composition that is specifically suitable for the treatment of ADHD, ADD, Asperger's syndrome and/or autism or for the amelioration or reduction of symptoms originating from ADHD, ADD, Asperger's syndrome and/or autism comprises *Lactobacillus plantarum* (LMG P-20606); *Lactobacillus paracasei* (LMG P-26118); *Pediococcus pentosacceus* (LMG P-20608); *Leuconostoc mesenterioides* (LMG P-20607); and *Bifidobacterium breve* (LMG P-26117).

Compositions that are specifically suitable for the treatment of ADHD, ADD, Asperger's syndrome and/or autism or for the amelioration or reduction of symptoms originating from ADHD, ADD, Asperger's syndrome and/or autism are Composition Nos. 19, 22, 37 and 40 in Table 1.

Examples of areas where the microbiota has been shown to play an important role is on various immune functions including inflammation and on the gut-brain axis. The gut-brain axis is the bidirectional interaction between the gut and the central nervus system. Molecules of importance for this interaction are e.g. short chain fatty acids (SCFAs) which can be produced by the gut microbiota if a suitable substrate is available e.g. soluble fibres or oligosaccharides. The most abundant short chain fatty acids (SCFAs) in stool and body fluids are formic acid, acetic acid, propionic acid and butyric acid. SCFAs are multifunctional molecules, being e.g. an essential energy source for local intestinal cells, but also influencing barrier function, neurotransmitter release, microglial maturation and activation, neural proliferation, mitochondrial function, oxidative stress, immune-modulation, and anti-inflammatory processes. SCFAs can regulate brain function and behavior via a number of potential mechanisms and dysregulation of these pathways may contribute to autism and related disorders. Butyric acid, formic acid, acetic acid and propionic acid have previously been reported to have anti-inflammatory effects. SCFAs regulate several leukocyte functions including production of cytokines, eicosanoids and chemokines.

Different immune markers have previously been studied in neuropsychiatric disorders. IL-12/IL-23p40 has been shown to be elevated in patients with schizophrenia. Thus, reducing the expression of IL-12/IL-23p40 is anticipated to have an effect on the clinical manifestation of this disease.

VCAM-1 is a cell surface glycoprotein expressed on e.g. endothelial cells and immune cells such as macrophages and dendritic cells. VCAM-1 was originally identified as a cell adhesion molecule that helps to regulate inflammation-associated vascular adhesion and the transendothelial migration of leukocytes, such as macrophages and T cells. Recent evidence suggests that VCAM-1 is closely associated with the progression of various immunological disorders.

ICAM-1 has been shown to be elevated in several neuropsychiatric disorders, e.g. ADHD, autism and Asperger's syndrome as well as borderline personality disorder, schizophrenia, depressive disorders, generalized anxiety disorder (GAD), and bipolar disorder. Thus, reducing the expression of ICAM-1 is anticipated to have an effect on the clinical manifestation of these diseases.

### Experiments and studies

### Immunological markers and short chain fatty acids in healthy individuals and individuals diagnosed with ADHD

The levels of selected immunological markers and short chain fatty acids were measured in blood samples from healthy individuals (n=57) and individuals diagnosed with ADHD (n=154, 8-55 years old, both men and women). A variety of immune activity markers of which some also are virus receptors were analysed.

As can be seen in Table 2, individuals diagnosed with ADHD have significantly (p < 0.005) lower expression of IL-10 (an anti-inflammatory marker) and significantly (p < 0.005) higher expression of the inflammatory markers sICAM-1and sVACM-1.

This clearly demonstrate that individuals diagnosed with ADHD express an inflammatory pattern regarding immune markers, and especially ICAM-1, which previously has been shown to be elevated in several neuropsychiatric disorders, e.g. ADHD, autism and Asperger's syndrome as well as borderline personality disorder, schizophrenia, depressive disorders, generalized anxiety disorder (GAD), and bipolar disorder.

Formic and propionic acids (short chain fatty acids) were lower in the individuals diagnosed with ADHD compared to the control group of 57 healthy individuals. Short chain fatty acids in plasma correlated positively with IL-10, i.e. individuals with a low level of short chain fatty acids also had a low level of the anti-inflammatory cytokine IL-10. This was seen both in children and in adults (data not shown).

**Table 2. Inflammatory activity markers/virus receptors, expressed in healthy individuals ("Control group") and individuals diagnosed with ADHD ("ADHD group")**

| **Marker** | **Control group Amount pg/ml (median)** | **ADHD group at baseline Amount pg/ml (median)** | **Significance, difference between control group and ADHD group (p)** |
|---|---|---|---|
| **IL-10** | 0.60 | 0.25 | 0.00202 |
| **sICAM-1** | 400,000 | 500,000 | 0.00202 |
| **sVCAM-1** | 400,000 | 480,000 | 0.00003 |

### Influence of a synbiotic composition corresponding to Composition No. 4 (not within the scope of the present claims) on inflammatory markers in individuals with ADHD

In the experiments described below, the effect of a synbiotic composition (corresponding to Composition No. 4) on the leves of i.a. IL-12/IL-23p40, ICAM-1, and SCFAs were studied.

A human intervention study was performed in a group of individuals (n=49, children, both boys and girls) diagnosed with ADHD. The effect of a synbiotic composition (corresponding to Composition No. 4) on plasma levels of immune markers and short fatty acids in children diagnosed with ADHD was investigated. The synbiotic composition included *Lactobacillus plantarum* LMG P-20606, *Lactobacillus paracasei* LMG P-26118, *Pediococcus pentosaceus* LMG P-20608 and the fibres inulin, pectin, resistant starch and beta-glucan. The total amount of bacteria was 4×10¹¹ and the total amount of fibres was 10g. The individuals diagnosed with ADHD were randomized into one group (n=28) receiving the synbiotic composition and one group (n=21) receiving a maltodextrin placebo. The synbiotic composition was consumed once daily for nine weeks. Immune markers were analysed in plasma at start of the study (T=0) and after nine weeks of intervention (T=9).

After 9 weeks, children receiving the synbiotic composition had a significantly (p<0.05) reduced level of the inflammatory marker IL-12/IL-23p40, sICAM and transforming growth factor β3 (TGF-β3) in plasma compared to the levels at start of the intervention study (Table 3).

The results show that the synbiotic composition lowered the expression of IL-12/IL-23p40, sICAM and TGF-β3 which all are inflammatory marker. IL-12/IL-23p40 is a target for drugs against inflammatory diseases, such as Crohn's disease and ICAM has been shown to be to be elevated in several neuropsychiatric disorders, e.g. ADHD, autism and Asperger's syndrome as well as borderline personality disorder, schizophrenia, depressive disorders, generalized anxiety disorder (GAD), and bipolar disorder.

**Table 3. Time effects over 9 weeks on inflammatory markers**

| **Marker** | **T=0 Amount pg/ml (median)** | **T=9 weeks Amount pg/ml (median)** | **Significance difference between T=0 and T=9 weeks (p)** |
|---|---|---|---|
| **IL-12/IL-23p40** | 260 | 238 | 0.0281 |
| **sICAM-1** | 740,000 | 670,000 | 0.0242 |
| **TGF-β3** | 4.1 | 3.6 | 0.0405 |

### Influence of a synbiotic composition (not within the scope of the present claims) on short chain fatty acids and their correlation to inflammatory markers

A human intervention study was performed in a group of individuals (n=49, children, both boys and girls) diagnosed with ADHD as described above. The synbiotic composition increased the levels of propionic and acetic acid, which correlated negatively to the levels of sICAM-1 and sVCAM-1, i.e. the proinflammatory markers were high when the short chain fatty acid level was low (data not shown). Thus, treatment with the synbiotic composition is associated with lower vascular inflammation. The results clearly show that the level of short chain fatty acids is associated to the level of immune markers, and with low levels of short chain fatty acids associated to a pro-inflammatory pattern of the immune markers. In the study it was also clear that the synbiotic composition increased the plasma level of short chain fatty acids which will stimulate a low inflammation. A low degree of inflammation is important for a healthy functioning of the gut-brain axis including neuropsychological conditions such as ADHD.

### Effect of a synbiotic composition according to the present invention (corresponding to Composition No. 19) on ADHD and/or borderline personality disorder (BPD)

To investigate the effect of a synbiotic composition corresponding to Composition No. 19 on reducing impulsive, compulsive, and aggressive behaviors in a sample of highly impulsive adults with a diagnosis of attention-deficit/hyperactivity disorder (ADHD) and/or borderline personality disorder (BPD), a 10-week multicenter, prospective, randomized, double-blind, placebo-controlled, parallel design study was performed. The composition comprised *Lactobacillus plantarum* (LMG P-20606), *Lactobacillus paracasei* (LMG P-17806), *Pediococcus pentosaceus* (LMG P-20608), and *Leuconostoc mesenteroides* (LMG P-20607) (total amount of bacteria 4×10¹¹ CFU/dose). Each dose further comprised inulin, pectin, resistant starch, beta-glucan (total amount of fibers per dose: 10g). The study was performed in close cooperation between three European clinical centers: Goethe University Hospital Frankfurt, Frankfurt, Germany; Vall d'Hebron Research Institute, Barcelona, Spain; and Semmelweis University, Budapest, Hungary.

### Study design

Participants: Participants were eligible for participation in the study if they met all the inclusion criteria and none of the exclusion criteria listed below. Participants were randomized to the experimental (EG) or the control (CG) group in a 1:1 allocation ratio (60 to 90 participants per group) using an independent web-based computerized service (www.randomization.com). Each individual received a "treatment kit" containing all required daily synbiotic or placebo envelope for the 10-week trial prepared in advance by sequentially numbering and labeled as "A" or "B." To secure bacterial viability, participants were asked to store the envelopes at + 4-6 °C. Participants were asked to continue with their previous medications and/or usual treatments and add the synbiotic or placebo once daily on top of cold foods such as yogurt, muesli, or salad.

Inclusion criteria: Both males and females aged 18 - 65 years; A high level of multidimensional impulsivity based on both a Clinical Global Impression-Severity Scale; (CGI-S) score ≥ 4 and an Affective Reactivity Index (ARI) ≥ 5; DSM-5 criteria for attention deficit/hyperactivity disorder (ADHD) and/or borderline personality disorder (BPD) confirmed by a structured diagnostic interview (ADHD: Diagnostic Interview for Adult ADHD [DIVA 2.0]; BPD: Structured Clinical Interview for DSM-IV [SCID-II]); Deemed reliable and compliant with the protocol by the investigator; Ability to speak and comprehend the native language of the country in which the assessments take place; Informed consent signed.

Exclusion criteria: Antibiotherapy within the last 6 weeks prior to study; Currently taking probiotics; Presence of a major psychiatric disorder with psychotic symptoms or other major psychiatric conditions requiring hospitalization (e.g., significant mood disorders); Neurological disorders involving central functions (e.g., epilepsy, multiple sclerosis, narcolepsy); Intelligence quotient (IQ) < 70 (measured by WAIS, if available); Major physical illnesses of the cardiovascular, endocrine, pulmonal, immune, or gastrointestinal system or undergoing immunosuppression; History of/present clinically relevant somatic acute or chronic disorders that, in the opinion of the investigator, might confound the results of tolerability/safety assessments or prohibit the patient from completing the study or would not be in the best interest of the patient; Documented allergy, hypersensitivity, or intolerance to any of the ingredients of the intervention; Subject has taken another investigational product or taken part in a clinical study within 30 days prior to entering the study.

Synbiotic composition: Each dose (powder-containing sachet) of the synbiotic composition contained 100 billion of each of *Pediococcus pentosaceus* (LMG P-20608), *Lactobacillus paracasei* (LMG P-17806), *Lactobacillus plantarum* (LMG P-20606), and *Leuconostoc mesenteroides* (LMG P-20607), in combination with four bioactive fermentable fibers (2.5 g each of β-glucan, inulin, pectin, and resistant starch). This composition corresponds to composition No. 19 in Table 1.

Placebo composition: The placebo was composed of the polysaccharide maltodextrin and had a texture and flavor similar to the synbiotic composition.

### Outcomes

Outcomes were assessed at the start of the study (TS), after 5 weeks (T1) and after 10 weeks (T2).

### Statistical analysis

Repeated measures ANCOVA with the factors Diagnosis (ADHD/BPD/ADHD+BPD) and Treatment group (Active/Placebo) and their interaction.

### Results

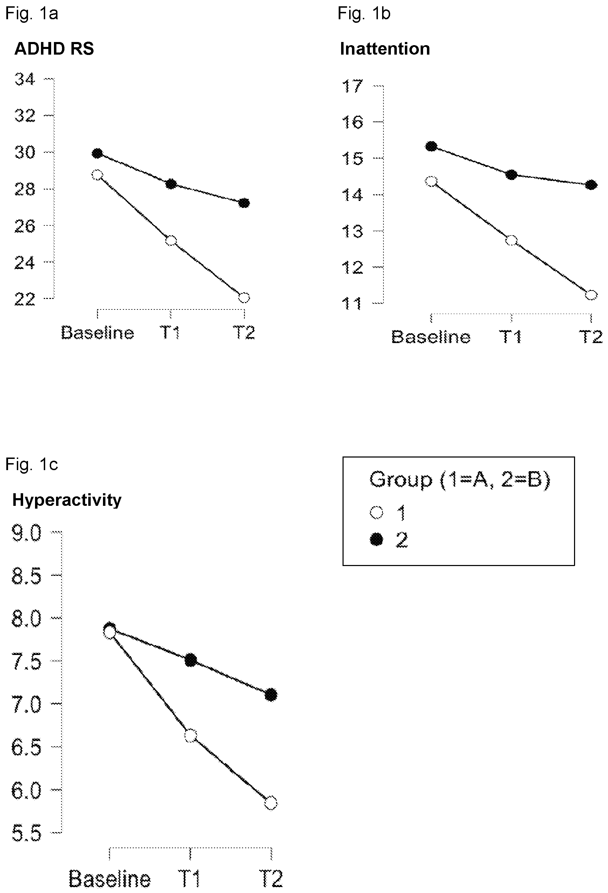

The results of this study is shown in Figures 1a to 1f. Open circles represent Group 1 = A = synbiotic composition) and filled circles represent Group 2 = B = placebo. Baseline = value/score at the beginning of the study; T1 = 5 weeks; T2 = 10 weeks. P-values are given for the difference between treated and untreated groups; p < 0.05 is considered as statistical significant; p < 0.1 is considered to represent a tendency of a difference between the treated and untreated groups. The data presented are data from LOCF (last observation carried forward) analyses.

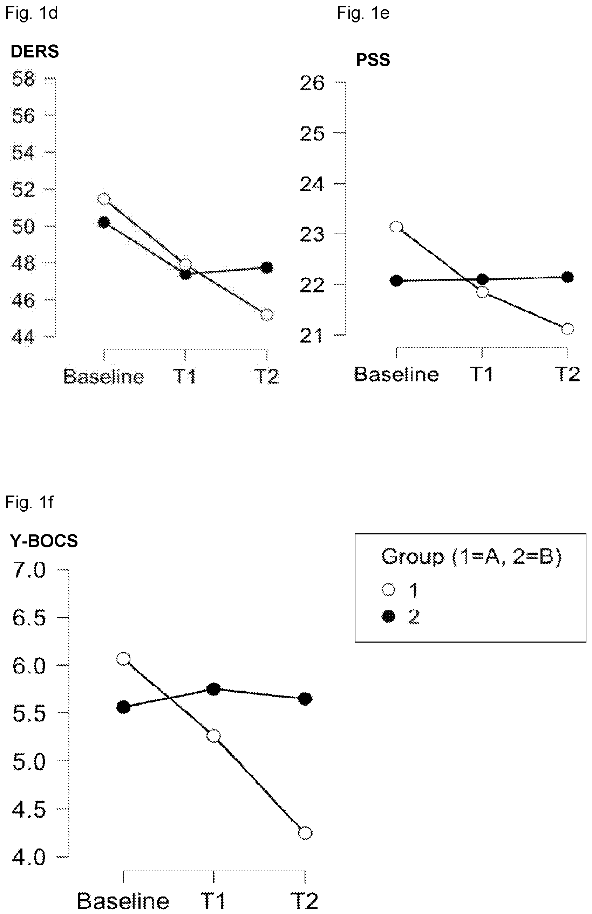

ADHD RS (Attention-Deficit/Hyperactivity Disorder Rating Scale): The ADHD-RS provides not only a total ADHD score (Fig. 1a), but also separate scores for inattentive (Fig. 1b), hyperactive (Fig. 1c), and impulsive subscales (Fig. 1d), providing a better characterization of patients. The ADHD-RS can be used to monitor changes in ADHD symptoms.

*Total score*: There was a dramatic decrease of ADHD symptoms [total score] (see Fig. 1a) in the group receiving the synbiotic composition (open circles; N=89 [55 ADHD; 23 BPD; 11 ADHD+BPD]) compared to the placebo group (filled circles; N=88 [56 ADHD; 20 BPD, 12 ADHD+BPD]). The difference was statistically significant (p = 0.038). Thus, a synbiotic composition according to the present disclosure reduces the symptoms of ADHD.

*Inattention:* There was also a decrease of inattention (see Fig. 1b) in the group receiving the synbiotic composition (open circles; N=90 [56 ADHD; 23 BPD; 11 ADHD+BPD]) compared to the placebo group (filled circles; N=88 [56 ADHD; 20 BPD, 12 ADHD+BPD]). The difference was statistically significant (p = 0.021). Thus, a synbiotic composition according to the present disclosure reduces inattention.

*Hyperactivity*: There was also a decrease of hyperactivity (see Fig. 1c) in the group receiving the synbiotic composition (open circles; N=90 [56 ADHD; 23 BPD; 11 ADHD+BPD]) compared to the placebo group (filled circles; N=88 [56 ADHD; 20 BPD, 12 ADHD+BPD]); but not statistically significant (p = 0.067). Thus, a synbiotic composition according to the present disclosure seems to reduce hyperactivity.

*Impulsivity*: No statistically significant decrease of impulsivity was observed in the group receiving the synbiotic composition (open circles; N=90 [56 ADHD; 23 BPD; 11 ADHD+BPD]) compared to the placebo group (filled circles; N=88 [56 ADHD; 20 BPD, 12 ADHD+BPD]). (Data not shown.)

*Conclusion*: Taken together, these results demonstrate that a synbiotic composition according to the present disclosure decreases at least inattention and hyperactivity. These symptoms are present in ADHD, but also in borderline personality disorder. ADHD or BDPD are often seen in patients with other disorders such as Asperger's syndrome, autism, borderline personality disorder, schizophrenia, depressive disorders, generalized anxiety disorder (GAD), and bipolar disorder.

DERS (Difficulties in Emotion Regulation Scale): DERS is a widely used self-report measure of subjective emotion ability, and is often used in treatment and research settings for adults with emotional (i.e., anxiety, mood, obsessive-compulsive, or trauma-related) disorders. Difficulties with emotion regulation are central to borderline personality disorder (BPD) and these difficulties are generally considered as difficult to treat with conventional medication.

*Total score*: There was a decrease of DERS [total score] (see Fig. 1d) in the group receiving the synbiotic composition (open circles; N=87 [55 ADHD; 21 BPD; 11 ADHD+BPD]) compared to the placebo group (filled circles; N=85 [54 ADHD; 19 BPD, 12 ADHD+BPD]); (p = 0.092). Thus, a synbiotic composition according to the present disclosure seems to reduce DERS.

*Conclusion*: These results demonstrate that a synbiotic composition according to the present disclosure decreases at least inattention and hyperactivity. These symptoms are present in ADHD, but also in borderline personality disorder and in autism. ADHD or BDPD are often seen in patients with other disorders such as Asperger's syndrome, autism, borderline personality disorder, schizophrenia, depressive disorders, generalized anxiety disorder (GAD), and bipolar disorder.

PSS (Perceived stress scale): The Perceived Stress Scale (PSS) is the most widely used psychological instrument for measuring the perception of stress. It is a measure of the degree to which situations in one's life are appraised as stressful.

*Total score*: There was also a decrease of perceived stress (see Fig. 1e) in the group receiving the synbiotic composition (open circles; N=90 [56 ADHD; 23 BPD; 11 ADHD+BPD]) compared to the placebo group (filled circles; N=88 [56 ADHD; 20 BPD, 12 ADHD+BPD]); The difference was statistically significant (p = 0.047). Thus, a synbiotic composition according to the present disclosure reduces perceived stress. Stress is present in basically all psychological disorders, such as ADHD, autism and borderline personality disorder, and also in Asperger's syndrome, schizophrenia, depressive disorders, generalized anxiety disorder (GAD), and bipolar disorder.

Y-BOCS (Yale-Brown Obsessive Compulsive Scale): Y-BOCS is a 10-item scale designed to measure the severity and type of symptoms in people with obsessive-compulsive disorder (OCD) over the past seven days. The symptoms assessed are obsessions and compulsions. Y-BOCS is regarded as the "gold standard" in assessing the severity of OCD symptoms and is widely used when tracking OCD symptoms at intake and during/after treatment.

Fig. 1f shows that obsessive compulsive symptoms decreased in the group receiving the synbiotic composition (open circles; N=89 [55 ADHD; 23 BPD; 11 ADHD+BPD]) compared to the placebo group (filled circles; N=88 [55 ADHD; 21 BPD, 12 ADHD+BPD]). (p = 0.087). Thus, a synbiotic composition according to the present disclosure seems to reduce obsessions and compulsions. Thus, a synbiotic composition according to the present disclosure can be used in reducing obsessive compulsive symptoms. Such symptoms are present in e.g. ADHD, borderline personality disorder, obsessive compulsive disorder (OCD), and can be present also in autism (includes Asberger), schizophrenia and GAD.

### Conclusions

ADHD/ADD: As shown above, symptoms associated ADHD, such as inattention, hyperactivity, difficulties in emotion regulation, perceived stress and obsessive compulsive behavior, can be affected by the intake of a synbiotic composition according to the present disclosure. Thus, it is plausible that a synbiotic composition according to the present disclosure can be used in the treatment of ADHD, although more research is needed for confirmation. As stated above, no statistically significant effect was seen on impulsivity. Thus, a synbiotic composition according to the present disclosure may particularly successfully used in the treatment of ADHD focusing on treatment of inattention. Specifically, a synbiotic composition according to the present disclosure may find use in the treatment of ADD.

Asperger's syndrome/Autism: As shown above, symptoms associated Asperger's syndrome/autism, such as inattention, perceived stress and obsessive compulsive symptoms, can be affected by the intake of a synbiotic composition according to the present disclosure. Thus, a synbiotic composition according to the present disclosure can be used in reducing symptoms of Asperger's syndrome/autism.

Borderline personality disorder: As shown above, symptoms associated with borderline personality disorder, such as difficulties in emotion regulation, perceived stress and obsessive compulsive symptoms, can be affected by the intake of the synbiotic composition. Thus, the synbiotic composition can be used in reducing symptoms in a patient suffering from borderline personality disorder (not within the scope of the present claims).

Obsessive compulsive disorder (OCD): The synbiotic composition seems to reduce obsessions and compulsions. Thus, the synbiotic composition can be used in reducing obsessive compulsive symptoms in a patient suffering from obsessive compulsive disorder (OCD) (not within the scope of the present claims).

Depressive disorders: As shown above, symptoms associated with depressive disorders, such as inattention, difficulties in emotion regulation and perceived stress, can be affected by the intake of the synbiotic composition. Thus, the synbiotic composition can be used in reducing symptoms in a patient suffering from depressive disorders (not within the scope of the present claims).

Generalized anxiety disorder: As shown above, symptoms associated with generalized anxiety disorder, such as difficulties in emotion regulation and perceived stress, can be affected by the intake of the synbiotic composition. Thus, the synbiotic composition can be used in reducing symptoms in a patient suffering from generalized anxiety disorder (not within the scope of the present claims).

Bipolar disorder: As shown above, symptoms associated with bipolar disorder, such as hyperactivity, difficulties in emotion regulation and perceived stress, can be affected by the intake of the synbiotic composition. Thus, the synbiotic composition can be used in reducing symptoms in a patient suffering from bipolar disorder (not within the scope of the present claims).

Comorbidity: Comorbidity with two or more of the above syndromes and disorders are common, partly due to overlapping symptoms. Thus, a synbiotic composition according to the present disclosure can be used in the treatment, amelioration and reduction of symptoms also in patients suffering from two or more of the syndromes and disorders above.

### User survey 1

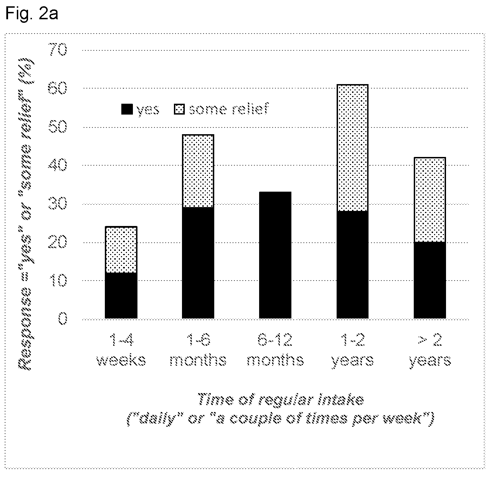

895 individuals (both men and women, from 18 years old) who had used a synbiotic composition corresponding to Composition No. 19 in Table 1 for 1 week up to 2 years, sporadically up to every day, were questioned about their experiences and perceived effects of intake of the composition. The composition comprised *Lactobacillus plantarum* (LMG P-20606), *Lactobacillus paracasei* (LMG P-17806), *Pediococcus pentosaceus* (LMG P-20608), and *Leuconostoc. mesenteroides* (LMG P-20607) (total amount of of bacteria was 1.5×10¹⁰ CFU/dose). Each dose further comprised inulin, pectin, resistant starch and beta-glucan (total amount of fibers 4 g). Each individual independently reported any experienced positive effect(s) as well as any negative effects. None reported severe adverse effect or that medical treatment was needed. 132 individuals reported that one purpose of using synbiotic composition was "for a good mental health". 109 of these respondents had used the synbiotic composition daily or a couple of times per week. Responses to the question whether they felt that the synbiotic composition had helped them with problems with depression/fatigue are shown in Table 4a and in Fig. 2a.

Notably, already after 1-4 weeks, 24% of the respondents reported that they had less problems with depression/fatigue and after 1-6 months, 48% of the respondents reported that they had less problems with depression/fatigue. After 1-2 years of regular intake, 61% of the respondents reported that they had less problems with depression/fatigue. The group "6-12 months" included 12 individuals, i.e. the smallest group in this survey and smaller than the groups "1-4 weeks", "1-6 months" and 1-2 years". Thus, in this group, a single individual's response will contribute to a larger extent to the percentage of reported effect of the synbiotic composition. Even though the percentage of responders reporting an effect on depression/fatigue is lower in the "more than 2 years"-group than in the "1-2 years"-group, still 42% of the respondents reported less problems with depression/fatigue. Thus, the results indicate that the synbiotic composition may reduce problems with depression/fatigue and can be used in the treatment of depressive disorders (not within the scope of the present claims).

**Table 4a. Number and percentage of individuals who reported effects of a synbiotic composition corresponding to Composition No. 19 in Table 1.**

| **Time period the synbiotic composition was used a couple of times per week or daily** | **Number of individuals** | **Do you feel that the synbiotic composition helped you with problems with depression/fatigue?** | |
|---|---|---|---|
| | | **Yes, absolutely** | **Yes,** I **get some relief** |
| **1-4 weeks** | 17 | 2 (12%) | 2 (12%) |
| **1-6 months** | 21 | 6 (29%) | 4 (19%) |
| **6-12 months** | 12 | 4 (33%) | 0 (0%) |
| **1-2 years** | 18 | 5 (28%) | 6 (33%) |
| **> 2 years** | 41 | 8 (20%) | 9 (22%) |

### User survey 2

93 individuals (both men and women, from 18 years old) who had used a synbiotic composition corresponding to Composition No. 19 in Table 1 for 4 weeks, sporadically up to every day, were questioned about their experiences and perceived effects of intake of the composition on effects concerning problems related to mental health. The composition comprised *Lactobacillus plantarum* (LMG P-20606), *Lactobacillus paracasei* (LMG P-17806), *Pediococcus pentosaceus* (LMG P-20608), and *Leuconostoc. mesenteroides* (LMG P-20607) (total amount of of bacteria was 1.5×10¹⁰ CFU/dose). Each dose further comprised inulin, pectin, resistant starch and beta-glucan (total amount of fibers 4 g). Each individual independently reported any experienced positive effect(s) as well as any negative effects. None reported severe adverse effect or that medical treatment was needed.13 individuals reported that they suffered from anxiety or other mental problems. 12 of these respondents had used the synbiotic composition daily, almost every day or about half of the days. Responses to the question whether they felt that the synbiotic composition had helped them with problems related to mental health are shown in Table 4b.

**Table 4b. Number and percentage of individuals (out of 12 individuals) who reported effects after 4 weeks of intake of a synbiotic composition corresponding to Composition No. 19 in Table 1.**

| **Problem** | **Do you feel that the synbiotic composition helped you with problems with [*problem*]?** | | |
|---|---|---|---|
| | **Yes, absolutely** | **Yes, I get some relief** | **Total percentage that perceived an effect** |
| **Fokus/concentration** | 0 (0%) | 5 (42%) | 42% |
| **Moodswings** | 0 (0%) | 5 (42%) | 42% |
| **Depression** | 0 (0%) | 4 (33%) | 33% |
| **Anxiety** | 1 (8%) | 3 (25%) | 33% |
| **Fatigue** | 0 (0%) | 3 (25%) | 25% |

As can be seen in Table 4b, already when the time period of regular intake of the synbiotic is as short as 4 weeks, respondents reported an improvement in symptoms such as focus/concentration, moodswings, depression, anxiety and fatigue. Thus, the results indicate that the synbiotic composition may reduce problems with these symptoms and can be used in the treatment of disorders such as depressive disorders, anxiety, such as generalized anxiety disorder and biopolar disorder (not within the scope of the present claims).

## Claims

1. A synbiotic composition for use in the treatment of a neuropsychiatric disorder or for use in the amelioration and/or reduction of symptoms originating from the neuropsychiatric disorder,
wherein the neuropsychiatric disorder is chosen from the group consisting of ADHD, ADD, Asperger's syndrome and autism; and
wherein the synbiotic composition comprises:
- *a Lactobacillus paracasei* strain, wherein the *Lactobacillus paracasei* strain is *Lactobacillus paracasei* (LMG P-17806) or *Lactobacillus paracasei* (LMG P-26118),
- *Pediococcus pentosaceus* (LMG P-20608),
- *Lactobacillus plantarum* (LMG P-20606), and
- *Leuconostoc mesenteroides* (LMG P-20607);
and at least one dietary fiber chosen from the group consisting of
- inulin,
- pectin,
- beta-glucan,
- resistant starch,
- a galacto-oligosaccharide,
- an isomalto-oligosaccharide, and
- a rice fiber.

2. The synbiotic composition for use according to claim 1, wherein the composition further comprises at least one *Bifidobacterium breve* strain, preferably *Bifidobacterium breve* (LMG P-26117).

3. The synbiotic composition for use according to any one of the preceding claims, wherein the *Lactobacillus paracasei* strain is *Lactobacillus paracasei* (LMG P-17806).

4. The synbiotic composition for use according to any one of the preceding claims, wherein the composition comprises inulin, pectin, beta-glucan and resistant starch.

5. The synbiotic composition for use according to any one of the preceding claims, wherein the composition comprises resistant starch, a galacto-oligosaccharide, and/or an isomalto-oligosaccharide.

6. The synbiotic composition for use according to any one of the preceding claims, wherein the symptoms of the neuropsychiatric disorder are chosen from the group consisting of:
- ADHD symptoms as measured by the Attention-Deficit/Hyperactivity Disorder Rating Scale),
- inattention, and
- hyperactivity.

7. The synbiotic composition for use according to any one of the preceding claims, wherein the symptoms of the neuropsychiatric disorder are chosen from the group consisting of:
- difficulty in emotion regulation,
- perceived stress, and
- obsessions and compulsions as measured by the Yale-Brown Obsessive Compulsive Scale.

8. The synbiotic composition for use according to any one of the preceding claims, wherein the total amount of bacteria in one dose is 1×10⁶ to 1×10¹³, such as 5×10⁶ to 1×10¹³, such as 1×10⁷ to 1×10¹³, such as 5×10⁷ to 1×10¹³, such as 1×10⁸ to 1×10¹³, such as 5×10⁸ to 5×10¹², especially 1×10⁹ to 1×10¹², such as 5×10⁹ to 9×10¹¹, such as 1×10¹⁰ to 8×10¹¹, such as 5×10¹⁰ to 7×10¹¹, such as 1×10¹¹ to 6×10¹¹, such as 3×10¹¹ to 5×10¹¹, such as 4×10¹¹ CFUs.

9. The synbiotic composition for use according to any one of the preceding claims, wherein the total amount of fibers in one dose is between 0.1 to 20 g, such as 0.5 to 20 g, such as 1 to 20 g, such as 2 to 15 g, such as 2.5 to 12.5 g, such as 5 to 10 g, such as 6 to 8.5 g, such as 7.5 g.

10. The synbiotic composition for use according to any one of the preceding claims, wherein the total amount of bacteria in one dose is 4×10¹¹ CFUs and the total amount of fibers in one dose is 10 g.

11. The synbiotic composition for use according to any one of the preceding claims, wherein the synbiotic composition is administered orally or rectally or by tube feeding.

12. The synbiotic composition for use according to any one of the preceding claims, wherein the synbiotic composition is administered 1 to 3 times a day, 1 to 7 times a week.

13. The synbiotic composition for use according to any one of the preceding claims, wherein the synbiotic composition is in the form of a powder, a capsule, a tablet, a lozenge, a liquid, an emulsion, an enema, a suppository, or a tube feeding; and/or wherein the synbiotic composition is a food supplement, a food product, a nutritional supplement, a natural remedy or a pharmaceutical product.

## Patentansprüche

1. Synbiotische Zusammensetzung zur Verwendung bei der Behandlung einer neuropsychiatrischen Störung oder zur Verwendung bei der Verbesserung und/oder Verringerung von Symptomen, die von der neuropsychiatrischen Störung herrühren,
wobei die neuropsychiatrische Störung ausgewählt ist aus der Gruppe bestehend aus ADHS, ADS, Asperger-Syndrom und Autismus; und
wobei die synbiotische Zusammensetzung umfasst:
- einen *Lactobacillus paracasei*-Stamm, wobei der *Lactobacillus paracasei*-Stamm *Lactobacillus paracasei* (LMG P-17806) oder *Lactobacillus paracasei* (LMG P-26118) ist,
- *Pediococcus pentosaceus* (LMG P-20608),
- *Lactobacillus plantarum* (LMG P-20606) und
- *Leuconostoc mesenteroides* (LMG P-20607);
und wenigstens einen Ballaststoff, ausgewählt aus der Gruppe bestehend aus
- Inulin,
- Pectin,
- Beta-Glucan,
- resistenter Stärke,
- einem Galactooligosaccharid,
- einem Isomaltooligosaccharid und
- einer Reisfaser.

2. Synbiotische Zusammensetzung zur Verwendung nach Anspruch 1, wobei die Zusammensetzung weiter wenigstens einen *Bifidobacterium* breve-Stamm umfasst, vorzugsweise *Bifidobacterium breve* (LMG P-26117).

3. Synbiotische Zusammensetzung zur Verwendung nach einem der vorangehenden Ansprüche, wobei der *Lactobaccilus paracasei*-Stamm *Lactobacillus paracasei* (LMG P-17806) ist.

4. Synbiotische Zusammensetzung zur Verwendung nach einem der vorangehenden Ansprüche, wobei die Zusammensetzung Inulin, Pectin, Beta-Glucan und resistente Stärke umfasst.

5. Synbiotische Zusammensetzung zur Verwendung nach einem der vorangehenden Ansprüche, wobei die Zusammensetzung resistente Stärke, ein Galactooligosaccharid und/oder ein Isomaltooligosaccharid umfasst.

6. Synbiotische Zusammensetzung zur Verwendung nach einem der vorangehenden Ansprüche, wobei die Symptome der neuropsychiatrischen Störung ausgewählt sind aus der Gruppe bestehend aus:
- ADHS-Symptomen, gemessen mit der Aufmerksamkeitsdefizit-/ Hyperaktivitätsstörung-Ratingskala,
- Unaufmerksamkeit und
- Hyperaktivität.

7. Synbiotische Zusammensetzung zur Verwendung nach einem der vorangehenden Ansprüche, wobei die Symptome der neuropsychiatrischen Störung ausgewählt sind aus der Gruppe bestehend aus:
- Schwierigkeiten bei der Emotionsregulation,
- wahrgenommenem Stress und
- Obsessionen und Kompulsionen, gemessen mit der Yale-Brown-Obsessiv-Kompulsiv-Skala.

8. Synbiotische Zusammensetzung zur Verwendung nach einem der vorangehenden Ansprüche, wobei die Gesamtmenge an Bakterien in einer Dosis 1×10⁶ bis 1×10¹³, wie etwa 5×10⁶ bis 1×10¹³, wie etwa 1×10⁷ bis 1×10¹³, wie etwa 5×10⁷ bis 1×10¹³, wie etwa 1×10⁸ bis 1×10¹³, wie etwa 5×10⁸ bis 5×10¹², insbesondere 1×10⁹ bis 1×10¹², wie etwa 5×10⁹ bis 9×10¹¹, wie etwa 1×10¹⁰ bis 8×10¹¹, wie etwa 5×10¹⁰ bis 7×10¹¹, wie etwa 1×10¹¹ bis 6×10¹¹, wie etwa 3×10¹¹ bis 5×10¹¹, wie etwa 4×10¹¹ KBEs beträgt.

9. Synbiotische Zusammensetzung zur Verwendung nach einem der vorangehenden Ansprüche, wobei die Gesamtmenge an Ballaststoffen in einer Dosis zwischen 0,1 bis 20 g, wie etwa 0,5 bis 20 g, wie etwa 1 bis 20 g, wie etwa 2 bis 15 g, wie etwa 2,5 bis 12,5 g, wie etwa 5 bis 10 g, wie etwa 6 bis 8,5 g, wie etwa 7,5 g beträgt.

10. Synbiotische Zusammensetzung zur Verwendung nach einem der vorangehenden Ansprüche, wobei die Gesamtmenge an Bakterien in einer Dosis 4×10¹¹ KBEs und die Gesamtmenge an Ballaststoffen in einer Dosis 10 g beträgt.

11. Synbiotische Zusammensetzung zur Verwendung nach einem der vorangehenden Ansprüche, wobei die synbiotische Zusammensetzung oral oder rektal oder durch Sondenernährung verabreicht wird.

12. Synbiotische Zusammensetzung zur Verwendung nach einem der vorangehenden Ansprüche, wobei die synbiotische Zusammensetzung 1- bis 3-mal täglich, 1- bis 7-mal wöchentlich verabreicht wird.

13. Synbiotische Zusammensetzung zur Verwendung nach einem der vorangehenden Ansprüche, wobei die synbiotische Zusammensetzung in der Form eines Pulvers, einer Kapsel, einer Tablette, einer Pastille, einer Flüssigkeit, einer Emulsion, eines Klistiers, eines Suppositoriums oder einer Sondenernährung vorliegt; und/oder wobei die synbiotische Zusammensetzung ein Nahrungsergänzungsmittel, ein Nahrungsmittelprodukt, ein Nährstoffergänzungsmittel, ein natürliches Heilmittel oder ein pharmazeutisches Produkt ist.

## Revendications

1. Composition synbiotique pour utilisation dans le traitement d'un trouble neuropsychiatrique ou pour utilisation dans l'amélioration et/ou la réduction de symptômes tirant son origine du trouble neuropsychiatrique,
dans laquelle le trouble neuropsychiatrique est choisi dans le groupe consistant en le TDAH (trouble déficitaire de l'attention avec hyperactivité), le TDA (trouble déficitaire de l'attention), le syndrome d'Asperger et l'autisme ; et
dans laquelle la composition synbiotique comprend :
- une souche de *Lactobacillus paracasei,* dans laquelle la souche de *Lactobacillus paracasei* est *Lactobacillus paracasei* (LMG P-17806) ou *Lactobacillus paracasei* (LMG P-26118),
- *Pediococcus pentosaceus* (LMG P-20608),
- *Lactobacillus plantarum* (LMG P-20606), et
- *Leuconostoc mesenteroides* (LMG P-20607) ;
et au moins une fibre alimentaire choisie dans le groupe consistant en
- l'inuline,
- la pectine,
- le bêta-glucane,
- l'amidon résistant,
- un galacto-oligosaccharide,
- un isomalto-oligosaccharide, et
- une fibre de riz.

2. Composition synbiotique pour utilisation selon la revendication 1, dans laquelle la composition comprend en outre au moins une souche de *Bifidobacterium breve,* de préférence *Bifidobacterium breve* (LMG P-26117).

3. Composition synbiotique pour utilisation selon l'une quelconque des revendications précédentes, dans laquelle la souche *Lactobacillus paracasei* est *Lactobacillus paracasei* (LMG P-17806).

4. Composition synbiotique pour utilisation selon l'une quelconque des revendications précédentes, dans laquelle la composition comprend de l'inuline, de la pectine, du bêta-glucane et de l'amidon résistant.

5. Composition synbiotique pour utilisation selon l'une quelconque des revendications précédentes, dans laquelle la composition comprend de l'amidon résistant, un galacto-oligosaccharide et/ou un isomalto-oligosaccharide.

6. Composition synbiotique pour utilisation selon l'une quelconque des revendications précédentes, dans laquelle les symptômes du trouble neuropsychiatrique sont choisis dans le groupe consistant en :
- symptômes de TDAH mesurés par l'échelle d'évaluation du trouble déficitaire de l'attention avec hyperactivité,
- inattention, et
- hyperactivité.

7. Composition synbiotique pour utilisation selon l'une quelconque des revendications précédentes, dans laquelle les symptômes du trouble neuropsychiatrique sont choisis dans le groupe consistant en :
- difficulté à réguler les émotions,
- stress perçu, et
- obsessions et compulsions telles que mesurées par l'échelle d'obsession-compulsion de Yale-Brown.

8. Composition synbiotique pour utilisation selon l'une quelconque des revendications précédentes, dans laquelle la quantité totale de bactéries dans une dose est de 1 x 10⁶ à 1 x 10¹³, telle que 5 x 10⁶ à 1 x 10¹³, telle que 1 x 10⁷ à 1 x 10¹³, telle que 5 x 10⁷ à 1 x 10¹³, telle que 1 x 10⁸ à 1 x 10¹³, telle que 5 x 10⁸ à 5 x 10¹², notamment 1 x 10⁹ à 1 x 10¹², telle que 5 x 10⁹ à 9 x 10¹¹, telle que 1 x 10¹⁰ à 8 x 10¹¹, telle que 5 x 10¹⁰ à 7 x 10¹¹, telle que 1 x 10¹¹ à 6 x 10¹¹, telle que 3 x 10¹¹ à 5 x 10¹¹, telle que 4 x 10¹¹ UFC.

9. Composition synbiotique pour utilisation selon l'une quelconque des revendications précédentes, dans laquelle la quantité totale de fibres dans une dose est entre 0,1 et 20 g, telle que 0,5 et 20 g, telle que 1 et 20 g, telle que 2 et 15 g, telle que 2,5 et 12,5 g, telle que 5 et 10 g, telle que 6 et 8,5 g, telle que 7,5 g.

10. Composition synbiotique pour utilisation selon l'une quelconque des revendications précédentes, dans laquelle la quantité totale de bactéries dans une dose est de 4 x 10¹¹ UFC et la quantité totale de fibres dans une dose est de 10 g.

11. Composition synbiotique pour utilisation selon l'une quelconque des revendications précédentes, dans laquelle la composition synbiotique est administrée par voie orale ou rectale ou par alimentation par sonde.

12. Composition synbiotique pour utilisation selon l'une quelconque des revendications précédentes, dans laquelle la composition synbiotique est administrée 1 à 3 fois par jour, 1 à 7 fois par semaine.

13. Composition synbiotique pour utilisation selon l'une quelconque des revendications précédentes, dans laquelle la composition synbiotique se présente sous la forme d'une poudre, d'une capsule, d'un comprimé, d'une pastille, d'un liquide, d'une émulsion, d'un lavement, d'un suppositoire, ou d'une alimentation par sonde ; et/ou dans laquelle la composition synbiotique est un complément alimentaire, un produit alimentaire, un complément nutritionnel, un remède naturel ou un produit pharmaceutique.
